(19) Europäisches Patentamt
European Patent Office
Office européen des brevets

(11) EP 1 482 798 B1

# (12) EUROPÄISCHE PATENTSCHRIFT

(45) Veröffentlichungstag und Bekanntmachung des Hinweises auf die Patenterteilung:
**24.05.2006 Patentblatt 2006/21**

(21) Anmeldenummer: **03743328.1**

(22) Anmeldetag: **26.02.2003**

(51) Int Cl.:
***A01N 47/24*** (2006.01) ***A01N 43/653*** (2006.01)
***A01N 43/40*** (2006.01) ***A01N 37/50*** (2006.01)
*A01N 43/653* (2006.01) *A01N 47/24* (2006.01)
*A01N 43/40* (2006.01) *A01N 37/50* (2006.01)

(86) Internationale Anmeldenummer:
**PCT/EP2003/001929**

(87) Internationale Veröffentlichungsnummer:
**WO 2003/073852 (12.09.2003 Gazette 2003/37)**

## (54) FUNGIZIDE MISCHUNG AUF DER BASIS VON PROTHIOCONAZOLE UND TRIFLOXYSTROBIN

FUNGICIDAL MIXTURE BASED ON PROTHIOCONAZOLE AND TRIFLOXYSTROBIN

MELANGE FONGICIDE A BASE DE PROTHIOCONAZOLE ET DE TRIFLOXYSTROBIN

(84) Benannte Vertragsstaaten:
**AT BE BG CH CY CZ DE DK EE ES FI FR GB GR HU IE IT LI LU MC NL PT SE SI SK TR**
Benannte Erstreckungsstaaten:
**LT LV RO**

(30) Priorität: **01.03.2002 DE 10208838**

(43) Veröffentlichungstag der Anmeldung:
**08.12.2004 Patentblatt 2004/50**

(60) Teilanmeldung:
**05026582.6 / 1 642 499**

(73) Patentinhaber: **BASF Aktiengesellschaft**
**67056 Ludwigshafen (DE)**

(72) Erfinder:
- **AMMERMANN, Eberhard**
**64646 Heppenheim (DE)**
- **STIERL, Reinhard**
**67112 Mutterstadt (DE)**
- **LORENZ, Gisela**
**67434 Neustadt (DE)**
- **STRATHMANN, Siegfried**
**67117 Limburgerhof (DE)**
- **SCHELBERGER, Klaus**
**67161 Gönnheim (DE)**
- **SPADAFORA, V. James**
**Sugar Land, TX 77478 (US)**
- **CHRISTEN, Thomas**
**67125 Dannstadt-Schauernheim (DE)**

(56) Entgegenhaltungen:
**EP-A- 0 460 575** **WO-A-00/63188**
**WO-A-96/16048** **WO-A-98/47367**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 482 798 B1

## Beschreibung

Fungizide Mischung, enthaltend

(1) 2-[2-(1-Chlorcyclopropyl)-3-(2-chlorphenyl)-2-hydroxypropyl]-2,4-dihydro-[1,2,4]-triazol-3-thion (Prothioconazole) der Formel I oder dessen Salze oder Addukte

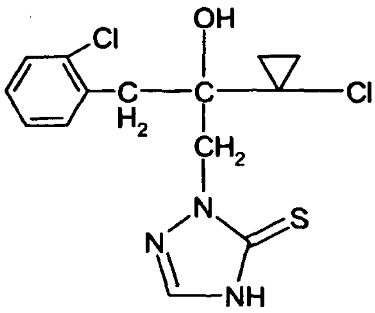

(I)

und

(2) Trifloxystrobin der Formel II

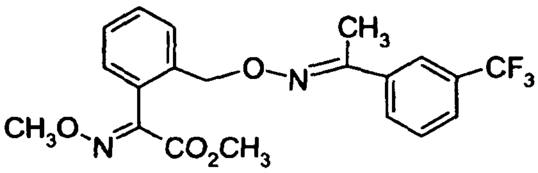

(II) (Trifloxystrobin)

(3)

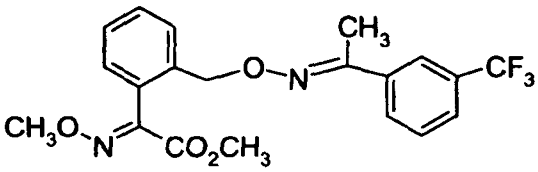

(II) (Trifloxystrobin)

oder dessen Salze oder Addukte in einer synergistisch wirksamen Menge.

Außerdem betrifft die Erfindung ein Verfahren zur Bekämpfung von Schadpilzen mit Mischungen der Verbindungen I mit der Verbindung II und die Verwendung der Verbindungen I und II zur Herstellung derartiger Mischungen sowie Mittel, die diese Mischungen enthalten.

Die Verbindung der Formel I, das 2-[2-(1-Chlorcyclopropyl)-3-(2-chlorphenyl)-2-hydroxypropyl]-2,4-dihydro-[1,2,4]-triazol-3-thion (Prothioconazol) ist bereits aus der WO 96/16048 bekannt.

Aus der WO 98/47367 ist eine Reihe von Wirkstoffkombinationen von Prothioconazol mit einer Vielzahl anderer fungizider Verbindungen bekannt.

Das Trifloxystrobin der Formel II und seine Verwendung als Pflanzenschutzmittel ist in der EP-A-0 460 575 beschrieben.

Im Hinblick auf eine Senkung der Aufwandmengen und eine Verbesserung des Wirkungsspektrums der bekannten Verbindungen I und II lagen der vorliegenden Erfindung Mischungen als Aufgabe zugrunde, die bei verringerter Gesamtmenge an ausgebrachten Wirkstoffen eine verbesserte Wirkung gegen Schadpilze aufweisen (synergistische Mischungen).

Demgemäß wurde die eingangs definierte Mischung von Prothioconazol mit einem Strobilurin-Derivat gefunden. Es wurde außerdem gefunden, daß sich bei gleichzeitiger, und zwar gemeinsamer oder getrennter Anwendung der Verbindung I und der Verbindung II oder der Verbindung I und der Verbindung II nacheinander Schadpilze besser bekämpfen lassen, als mit den Einzelverbindungen allein.

Das 2-[2-(1-Chlorcyclopropyl)-3-(2-chlorphenyl)-2-hydroxypropyl]-2,4-dihydro-[1,2,4]-triazol-3-thion der Formel I ist aus der WO 96-16 048 bekannt. Die Verbindung kann in der "Thiono"-Form der Formel

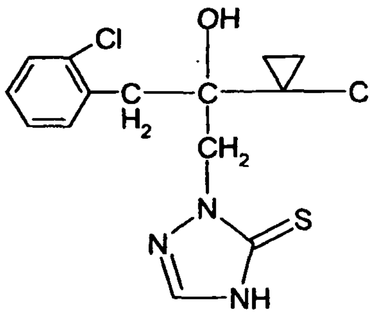

**(I)**

oder in der tautomeren "Mercapto"-Form der Formel

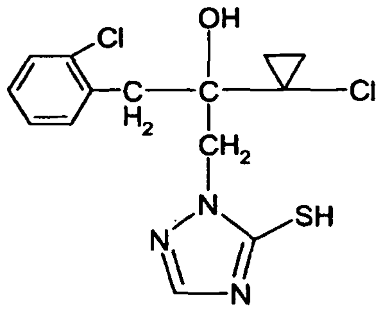

**(Ia)**

vorliegen. Der Einfachheit halber wird jeweils nur die "Thiono"-Form aufgeführt.

Das Trifloxystrobin der Formel II

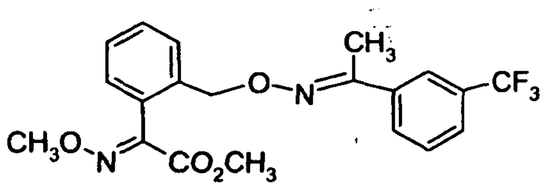

**(II) (Trifloxystrobin)**

ist aus der EP-A 0 460 572 bekannt.

Die Verbindungen I bis II sind wegen des basischen Charakters der in ihnen enthaltenen Stickstoffatome in der Lage, mit anorganischen oder organischen Säuren oder mit Metallionen Salze oder Addukte zu bilden.

Beispiele für anorganische Säuren sind Halogenwasserstoffsäuren wie Fluorwasserstoff, Chlorwasserstoff, Bromwasserstoff und Jodwasserstoff, Schwefelsäure, Phosphorsäure und Salpetersäure.

Als organischen Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bemsteinsäure, Zitronensäure, Benzoesäure, Zimtsäure, Oxalsäure, Alkylsulfonsäuren (Sulfonsäuren mit geradkettigen oder verzweigten Alkylresten mit 1 bis 20 Kohlenstoffatomen), Arylsulfonsäuren oder - disulfonsäuren (aromatische Reste wie Phenyl und Naphthyl welche eine oder zwei Sulfonsäuregruppen tragen), Alkylphosphonsäuren (Phosphonsäuren mit geradkettigen oder verzweigten Alkylresten mit 1 bis 20 Kohlenstoffatomen), Arylphosphonsäuren oder -diphosphonsäuren (aromatische Reste wie Phenyl und Naphthyl welche eine oder zwei Phosphorsäurereste tragen), wobei die Alkyl- bzw. Arylreste weitere Substituenten tragen können, z.B. p-Toluolsulfonsäure, Salizylsäure, p-Aminosalizylsäure, 2-Phenoxybenzoesäure, 2-Acetoxybenzoesäure etc.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der Nebengruppen der vierten Periode. Die Metalle können dabei in den verschiedenen ihnen zukommenden Wertigkeiten vorliegen.

Bevorzugt setzt man bei der Bereitstellung der Mischungen die reinen Wirkstoffe I und II ein, denen man weitere Wirkstoffe gegen Schadpilze oder gegen andere Schädlinge wie Insekten, Spinntiere oder Nematoden oder auch herbizide oder wachstumsregulierende Wirkstoffe oder Düngemittel beimischen kann.

Die Mischungen aus der Verbindung I mit der Verbindung II bzw. die Verbindung I mit der Verbindung II gleichzeitig, gemeinsam oder getrennt angewandt, zeichnen sich durch eine hervorragende Wirkung gegen ein breites Spektrum von pflanzenpathogenen Pilzen, insbesondere aus der Klasse der Ascomyceten, Basidiomyceten, Phycomyceten und Deuteromyceten aus. Sie sind z.T. systemisch wirksam und können daher auch als Blatt- und Bodenfungizide eingesetzt werden.

Besondere Bedeutung haben sie für die Bekämpfung einer Vielzahl von Pilzen an verschiedenen Kulturpflanzen wie Baumwolle, Gemüsepflanzen (z.B. Gurken, Bohnen, Tomaten, Kartoffeln und Kürbisgewächse), Gerste, Gras, Hafer, Bananen, Kaffee, Mais, Obstpflanzen, Reis, Roggen, Soja, Wein, Weizen, Zierpflanzen, Zuckerrohr sowie an einer Vielzahl von Samen.

Insbesondere eignen sie sich zur Bekämpfung der folgenden pftanzenpathogenen Pilze: *Blumeria graminis* (echter Mehltau) an Getreide, *Erysiphe cichoracearum* und *Sohaerotheca fuliginea* an Kürbisgewächsen, *Podosphaera leucotricha* an Apfeln, *Uncinula necator* an Reben, *Puccinia*-Arten an Getreide, *Rhizoctonia*-Arten an Baumwolle, Reis und Rasen, *Ustilago*-Arten an Getreide und Zuckerrohr, *Venturia inaequalis* (Schorf) an Äpfeln, *Helminthosporium*-Arten an Getreide, *Septoria nodorum* an Weizen, *Botrytis cinera* (Grauschimmel) an Erdbeeren, Gemüse, Zierpflanzen und Reben, *Cercospora arachidicola* an Erdnüssen, *Pseudocercosporella herpotrichoides* an Weizen und Gerste, *Pyricularia oryzae* an Reis, *Phytophthora infestans* an Kartoffeln und Tomaten, Plasm'opara viticola an Reben, *Pseudoperonospora*-Arten in Hopfen und Gurken, *Alternaria*-Arten an Gemüse und Obst, *Mycosphaerella*-Arten in Bananen sowie *Fusarium*- und *Verticillium*-Arten.

Sie sind außerdem im Materialschutz (z.B. Holzschutz) anwendbar, beispielsweise gegen Paecilomyces variotii.

Die Verbindung I mit der Verbindung II können gleichzeitig, und zwar gemeinsam oder getrennt, oder nacheinander aufgebracht werden, wobei die Reihenfolge bei getrennter Applikation im allgemeinen keine Auswirkung auf den Bekämpfungserfolg hat.

Die Verbindungen I und II werden üblicherweise in einem Gewichtsverhältnis von 20:1 bis 1:20, insbesondere 10:1 bis 1:10, vorzugsweise 5:1 bis 1:5 angewendet.

Die Aufwandmengen der erfindungsgemäßen Mischungen liegen, vor allem bei landwirtschaftlichen Kulturflächen, je nach Art des gewünschten Effekts bei 0,01 bis 8 kg/ha, vorzugsweise 0,1 bis 5 kg/ha, insbesondere 0,1 bis 3,0 kg/ha.

Die Aufwandmengen liegen dabei für die Verbindung I bei 0,01 bis 1 kg/ha, vorzugsweise 0,05 bis 0,5 kg/ha, insbesondere 0,05 bis 0,3 kg/ha.

Die Aufwandmengen für die Verbindung II liegen entsprechend bei 0,01 bis 1 kg/ha, vorzugsweise 0;02 bis 0,5 kg/ha, insbesondere 0,05 bis 0,3 kg/ha.

Bei der Saatgutbehandlung werden im allgemeinen Aufwandmengen an Mischung von 0,001 bis 250 g/kg Saatgut, vorzugsweise 0,01 bis 100 g/kg, insbesondere 0,01 bis 50 g/kg verwendet.

Sofern für Pflanzen pathogene Schadpilze zu bekämpfen sind, erfolgt die getrennte oder gemeinsame Applikation der Verbindung I mit der Verbindung II oder der Mischungen aus der Verbindung I mit der Verbindung II durch Besprühen oder Bestäuben der Samen, der Pflanzen oder der Böden vor oder nach der Aussaat der Pflanzen oder vor oder nach dem Auflaufen der Pflanzen.

Die erfindungsgemäßen fungiziden synergistischen Mischungen bzw. die Verbindung I und die Verbindung II können beispielsweise in Form von direkt versprühbaren Lösungen, Pulver und Suspensionen oder in Form von hochprozentigen wäßrigen, öligen oder sonstigen Suspensionen, Dispersionen, Emulsionen, Öldispersionen, Pasten, Stäubemitteln, Streumitteln oder Granulaten aufbereitet und durch Versprühen, Vemebeln, Verstäuben, Verstreuen oder Gießen angewendet werden. Die Anwendungsform ist abhängig vom Verwendungszweck; sie soll in jedem Fall eine möglichst feine und gleichmäßige Verteilung der erfindungsgemäßen Mischung gewährleisten.

Die Formulierungen werden in an sich bekannter Weise hergestellt, z.B. durch Zugabe von Lösungsmitteln und/oder Trägerstoffen. Den Formulierungen werden üblicherweise inerte Zusatzstoffe wie Emulgiermittel oder Dispergiermittel beigemischt.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanole oder Fettalkoholglycolethem, Kondensationsprodukte von sulfoniertem Naphthalin und seinen Derivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol- oder Tributylphenylpolyglycolether, Alkylarylpolyetheralkohole, Isotridecylalkohol; Fettalkoholethylenoxid-Kondensate, ethoxyliertes Rizinusöl, Polyoxyethylenalkylether oder Polyoxypropylen, Laurylalkoholpolyglycoletheracetat, Sorbitester, Lignin-Sulfitablaugen oder Methylcellulose in Betracht.

Pulver Streu- und Stäubemittel können durch Mischen oder gemeinsames Vermahlen der Verbindung I und der Verbindung II oder der Mischung aus der Verbindung I mit der Verbindung II mit einem festen Trägerstoff hergestellt werden.

Granulate (z.B. Umhüllungs-, Imprägnierungs- oder Homogengranulate) werden üblicherweise durch Bindung des Wirkstoffs oder der Wirkstoffe an einen festen Trägerstoff hergestellt.

Als Füllstoffe bzw. feste Trägerstoffe dienen beispielsweise Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, sowie Düngemittel wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulienrngen enthalten im allgemeinen 0,1 bis 95 Gew.-%, vorzugsweise 0,5 bis 90 Gew.-% der Verbindung I und der Verbindung II bzw. der Mischung aus der Verbindung I mit der Verbindung II. Die Wirkstoffe werden dabei in einer Reinheit von 90% bis 100%, vorzugsweise 95% bis 100% (nach NMR- oder HPLC-Spektrum) eingesetzt.

Die Anwendung der Verbindung I und der Verbindung II oder der Mischungen oder der entsprechenden Formulierungen erfolgt so, daß man die Schadpilze, deren Lebensraum oder die von ihnen freizuhaltenden Pflanzen, Samen, Böden, Flächen, Materialien oder Räume mit einer fungizid wirksamen Menge der Mischung, bzw. der Verbindung I und der Verbindung II bei getrennter Ausbringung, behandelt.

Die Anwendung kann vor oder nach dem Befall durch die Schadpilze erfolgen.

Anwendungsbeispiel

Die synergistische Wirkung der erfindungsgemäßen Mischungen ließ sich durch die folgenden Versuche zeigen:

Die Wirkstoffe wurden getrennt oder gemeinsam als 10%ige Emulsion in einem Gemisch aus 63 Gew.-% Cyclohexanon und 27 Gew.-% Emulgator aufbereitet und entsprechend der gewünschten Konzentration mit Wasser verdünnt.

Die Auswertung erfolgte durch Feststellung der befallenen Blattflächen in Prozent. Diese Prozent-Werte wurden in Wirkungsgrade umgerechnet. Der Wirkungsgrad (**W**) wurde nach der Formel von Abbot wie folgt bestimmt:

$$W = \left(1 - \frac{\alpha}{\beta}\right) \cdot 100$$

α entspricht dem Pilzbefall der behandelten Pflanzen in % und

β entspricht dem Pilzbefall der unbehandelten (Kontroll-) Pflanzen in %

Bei einem Wirkungsgrad von 0 entspricht der Befall der behandelten Pflanzen demjenigen der unbehandelten Kontrollpflanzen; bei einem Wirkungsgrad von 100 wiesen die behandelten Pflanzen keinen Befall auf.

Die zu erwartenden Wirkungsgrade der Wirkstoffmischungen wurden nach der Colby Formel [R.S. Colby, Weeds 15, 20-22 (1967)] ermittelt und mit den beobachteten Wirkungsgraden verglichen.

$$\text{Colby Formel: } E = x + y - x \cdot y / 100$$

E zu erwartender Wirkungsgrad, ausgedrückt in % der unbehandelten Kontrolle, beim Einsatz der Mischung aus den Wirkstoffen A und B in den Konzentrationen a und b

x der Wirkungsgrad, ausgedrückt in % der unbehandelten Kontrolle, beim Einsatz des Wirkstoffs A in der Konzentration a

y der Wirkungsgrad, ausgedrückt in % der unbehandelten Kontrolle, beim Einsatz des Wirkstoffs B in der Konzentration b

Anwendungsbeispiel 1: Wirksamkeit gegen Weizenmehltau verursacht durch *Erysiphe [syn. Blumeria] graminis* forma specialis. *tritici*

Blätter von in Töpfen gewachsenen Weizenkeimlingen der Sorte "Kanzier" wurden mit wäßriger Wirkstoffaufbereitung, die aus einer Stammlösung bestehend aus 10 % Wirkstoff, 85 % Cyclohexanon und 5 % Emulgiermittel angesetzt wurde, bis zur Tropfnässe besprüht und 24 Stunden nach dem Antrocknen des Spritzbelages mit Sporen des Weizenmehltaus *(Erysiphe [syn. Blumeria] graminis* forma specialis. tritici) bestäubt. Die Versuchspflanzen wurden anschließend im Gewächshaus bei Temperaturen zwischen 20 und 24°C und 60 bis 90 % relativer Luftfeuchtigkeit aufgestellt. Nach 7 Tagen wurde das Ausmaß der Mehltauentwicklung visuell in % Befall der gesamten Blattfläche ermittelt.

Die visuell ermittelten Werte für den Prozentanteil befallener Blattflächen wurden in Wirkungsgrade als % der unbehandelten Kontrolle umgerechnet. Wirkungsgrad 0 ist gleicher Befall wie in der unbehandelten Kontrolle, Wirkungsgrad 100 ist 0 % Befall. Die zu erwartenden Wirkungsgrade für Wirkstoffkombinationen wurden nach der obengenannten Colby-Formel ermittelt und mit den beobachteten Wirkungsgraden verglichen.

Tabelle 1

| Wirkstoff | Wirkstoffkonzentration in der Spritzbrühe in ppm | Wirkungsgrad in % der unbehandelten Kontrolle |
|---|---|---|
| Kontrolle (unbehandelt) | (90 % Befall) | 0 |
| Verbindung I = Prothiocoriazol | 4<br>1<br>0.25<br>0,06<br>0,015 | 22<br>0<br>0<br>0<br>0 |
| Verbindung II = Trifloxystrobin | 4<br>1<br>0.25<br>0,06 | 83<br>44<br>22<br>0 |

Tabelle 2

| Erfindungsgemäße Kombinationen | Beobachteter Wirkungsgrad | Berechneter Wirkungsgrad*) |
|---|---|---|
| Verbindung I = Prothioconazol + Verbindung II = Trifloxystrobin 0,015+0,25 ppm<br>Mischung 1 : 16 | 33 | 22 |
| Verbindung I = Prothioconazol + Verbindung II = Trifloxystrobin 1 : 4 ppm<br>Mischung 1 : 4 | 94 | 83 |
| Verbindung I = Prothioconazol + Verbindung II = Trifloxystrobin 0,25 + 1 ppm<br>Mischung 1 : 4 | 56 | 44 |
| Verbindung I = Prothioconazol + Verbindung II = Trifloxystrobin 0,25+0,06 ppm Mischung 4 : 1 | 22 | 0 |
| Verbindung I = Prothioconazol + Verbindung II = Trifloxystrobin 4 + 0,25 | 55 | 40 |

Tabelle fortgesetzt

| Erfindungsgemäße Kombinationen | Beobachteter Wirkungsgrad | Berechneter Wirkungsgrad*) |
|---|---|---|
| ppm<br>Mischung 16 : 1 | | |
| *) berechnet nach der Colby-Formel | | |

Aus den Ergebnissen des Versuches geht hervor, daß der beobachteter Wirkungsgrad in allen Mischungsverhältnissen höher ist, als nach der Colby-Fornnel vorausberechnete Wirkungsgrad (aus Synerg 171. XLS).

Anwendungsbeispiel 2: Kurative Wirksamkeit gegen Weizenbraunrost verursacht durch *Puccinia recondita*

Blätter von in Töpfen gewachsenen Weizensämlingen der Sorte "Kanzler" wurden mit Sporen des Braunrostes *(Puccinia recondita)* bestäubt. Danach wurden die Töpfe für 24 Stunden in eine Kammer mit hoher Luftfeuchtigkeit (90 bis 95 %) und 20 bis 22_ C gestellt. Während dieser Zeit keimten die Sporen aus und die Keimschläuche drangen in das Blattgewebe ein. Die infizierten Pflanzen wurden am nächsten Tag mit einer wäßrigen Wirkstoffaufbereitung, die aus einer Stammlösung bestehend aus 10 % Wirkstoff, 85 % Cyclohexanon und 5 % Emulgiermittel angesetzt wurde, tropfnaß besprüht. Nach dem Antrocknen des Spritzbelages wurden die Versuchspflanzen im Gewächshaus bei Temperaturen zwischen 20 und 22_ C und 65 bis 70 % relativer Luftfeuchte für 7 Tage kultiviert. Dann wurde das Ausmaß der Rostpilzentwicklung auf den Blättern ermittelt.

Die visuell ermittelten Werte für den Prozentanteil befallener Blattflächen wurden in Wirkungsgrade als % der unbehandelten Kontrolle umgerechnet. Wirkungsgrad 0 ist gleicher Befall wie in der unbehandelten Kontrolle, Wirkungsgrad 100 ist 0 % Befall. Die zu erwartenden Wirkungsgrade für Wirkstoffkombinationen wurden nach der obengenannten Colby-Formel ermittelt und mit den beobachteten Wirkungsgraden verglichen.

Tabelle 3

| Wirkstoff | Wirkstoffkonzentration in der Spritzbrühe in ppm | Wirkungsgrad in % der unbehandelten Kontrolle |
|---|---|---|
| Kontrolle (unbehandelt) | (90 % Befall) | 0 |
| Verbindung I = Prothioconazol | 1<br>0.25<br>0,015<br>0,006 | 0<br>0<br>0<br>0 |
| Verbindung II = Trifloxystrobin | 0.25<br>0,06 | 0<br>0 |

Tabelle 4

| Erfindungsgemäße Kombinationen | Beobachteter Wirkungsgrad | Berechneter Wirkungsgrad*) |
|---|---|---|
| Verbindung I = Prothioconazol + Verbindung II = Trifloxystrobin 0,015+0,25 ppm Mischung 1 : 16 | 22 | 0 |
| Verbindung I = Prothioconazol + Verbindung II = Trifloxystrobin 0,06 : 0,25 ppm<br>Mischung 1 : 4 | 22 | 0 |
| Verbindung I = Prothioconazol + Verbindung II = Trifloxystrobin 1 + 0,25 ppm<br>Mischung 4 : 1 | 67 | 0 |

Tabelle fortgesetzt

| Erfindungsgemäße Kombinationen | Beobachteter Wirkungsgrad | Berechneter Wirkungsgrad*) |
|---|---|---|
| Verbindung I = Prothioconazol + Verbindung II = Trifloxystrobin 0,25 + 0,06 ppm<br>Mischung 4 : 1 | 67 | 0 |
| Verbindung I = Prothioconazol + Verbindung II = Trifloxystrobin 1 + 0,06 ppm<br>Mischung 16 : 1 | 11 | 0 |
| *) berechnet nach der Colby-Formel | | |

Aus den Ergebnissen des Versuches geht hervor, daß der beobachteter Wirkungsgrad in allen Mischungsverhältnissen höher ist, als nach der Colby-Formel vorausberechnete Wirkungsgrad (aus Synerg 171. XLS).

## Patentansprüche

1. Fungizide Mischung, enthaltend

(1) 2-[2-(1-Chlorcyclopropyl)-3-(2-chlorphenyl)-2-hydroxypropyl]-2,4-dihydro-[1,2,4]-triazol-3-thion (Prothioconazole) der Formel I oder dessen Salze oder Addukte

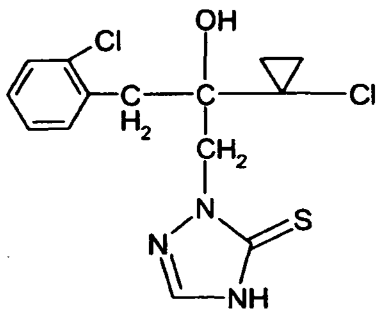

(I)

und
(2) Trifloxystrobin der Formel II

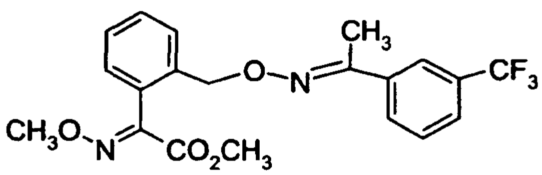

**(II) (Trifloxystrobin)**

oder dessen Salze oder Addukte
in einer synergistisch wirksamen Menge.

2. Fungizide Mischung nach Anspruch 1, **dadurch gekennzeichnet, daß** das Gewichtsverhältnis von Prothioconazole der Formel I zu Trifloxystrobin der Formel II 20:1 bis 1:20 beträgt.

3. Verfahren zur Bekämpfung von Schadpilzen, **dadurch gekennzeichnet, daß** man die Schadpilzen, deren Lebens-

raum oder die von ihnen freizuhaltenden Pflanzen, Samen, Böden, Flächen, Materialien oder Räume mit der fungiziden Mischung gemäß Anspruch 1 behandelt.

4. Verfahren nach Anspruch 3, **dadurch gekennzeichnet, daß** man die Verbindung der Formel I gemäß Anspruch 1 und eine Verbindung der Formel II gemäß Anspruch 1 gleichzeitig, und zwar gemeinsam oder getrennt, oder nacheinander ausbringt.

5. Verfahren nach Anspruch 3 oder 4, **dadurch gekennzeichnet, daß** man die fungizide Mischung oder die Verbindung der Formel I mit einer Verbindung der Formel II gemäß Anspruch 1 in einer Menge von 0,01 bis 8 kg/ha aufwendet.

6. Fungizide Mittel, enthaltend die fungizide Mischung gemäß Anspruch 1 sowie einen festen oder flüssigen Träger.

**Claims**

1. A fungicidal mixture, comprising

(1) 2-[2-(l-chlorocyclopropyl)-3-(2-chlorophenyl)-2-hydroxypropyl]-2,4-dihydro-[1,2,4]-triazole-3-thione (prothioconazole) of the formula I or its salts or adducts

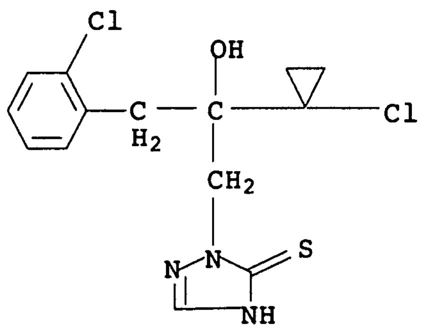

(I)

and
(2) trifloxystrobin of the formula II

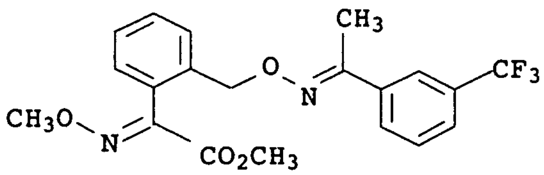

(II) (Trifloxystrobin)

or its salts or adducts
in a synergistically effective amount.

2. The fungicidal mixture according to claim 1, wherein the weight ratio of prothioconazole of the formula I to trifloxystrobin of the formula II is from 20:1 to 1:20.

3. A method for controlling harmful fungi, which comprises treating the harmful fungi, their habitat or the plants, seeds, soils, areas, materials or spaces to be kept free from them with the fungicidal mixture according to claim 1.

4. The method according to claim 3, wherein the compound of the formula I according to claim 1 and a compound of the formula II according to claim 1 are applied simultaneously, that is together or separately, or in succession.

5. The method according to claim 3 or 4, wherein the fungicidal mixture or the compound of the formula I with a compound of the formula II according to claim 1 is applied in an amount of from 0.01 to 8 kg/ha.

**6.** A fungicidal composition, comprising the fungicidal mixture according to claim 1 and a solid or liquid carrier.

## Revendications

**1.** Mélange fongicide, contenant

(1) de la 2-[2-(1-chlorocyclopropyl)-3-(2-chlorophênyl)-2-hydroxypropyl]-2,4-dihydro-[1,2,4]-triazol-3-thione (prothioconazole) de la formule I ou ses sels ou produits d'addition :

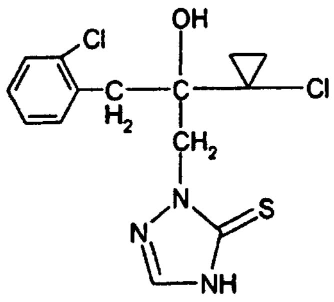

(I)

et

(2) du trifloxystrobin de la formule II :

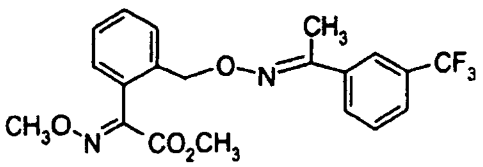

(II) (trifloxystrobin)

ou ses sels ou produits d'addition,
en une quantité efficace du point de vue synergique.

**2.** Mélange fongicide suivant la revendication 1, **caractérisé en ce que** le rapport pondéral entre le prothioconazole de la formule I et le trifloxystrobin de la formule II est de 20/1 à 1/20.

**3.** Procédé pour lutter contre des champignons nuisibles, **caractérisé en ce qu'**on traite les champignons nuisibles, leur environnement ou les plantes, semences, sols, surfaces, matériels ou espaces dont il faut les libérer, avec le mélange fongicide suivant la revendication 1.

**4.** Procédé suivant la revendication 3, **caractérisé en ce qu'**on répand le composé de la formule I suivant la revendication 1 et un composé de la formule II suivant la revendication 1 simultanément, et cela conjointement ou séparément, ou successivement.

**5.** Procédé suivant la revendication 3 ou 4, **caractérisé en ce qu'**on applique le mélange fongicide ou le composé de la formule I avec un composé de la formule II suivant la revendication 1, en une quantité de 0,01 à 8 kg/ha.

**6.** Produit fongicide, contenant le mélange fongicide suivant la revendication 1 ainsi qu'un support solide ou liquide.